# EUROPEAN PATENT APPLICATION

(11) **EP 4 083 650 A1**
(43) Date of publication of application: **02.11.2022**
(21) Application number: 21170410.1
(22) Date of filing: 26.04.2021
(51) Int. Cl.: G01R 33/48, A61B 6/00, A61B 8/08, G06T 7/00

(54) **CONTROLLING A SCANNING OPERATION OF A MEDICAL IMAGING DEVICE**

(71) Applicant: Koninklijke Philips N.V., 5656 AG Eindhoven (NL)
(72) Inventor: DEGERHOLM, Markku Aleksanteri, 5656 AE Eindhoven (NL); GROTH, Alexandra, 5656 AE Eindhoven (NL); SOMMER, Karsten, 5656 AE Eindhoven (NL); SUSI, Tuomas, 5656 AE Eindhoven (NL); WEESE, Rolf Jürgen, 5656 AE Eindhoven (NL); WAECHTER-STEHLE, Irina, 5656 AE Eindhoven (NL); SAALBACH, Axel, 5656 AE Eindhoven (NL)
(74) Representative: Philips Intellectual Property & Standards

(57) **Abstract**

An approach for adapting the scanning operation of a medical imaging device so that anatomical areas of interest are scanned with a higher image quality than other anatomical regions. Initial image data is processed to identify relevant anatomical areas or regions, by identifying a likelihood that these areas contain a pathological condition/symptom. The scanning operation is controlled based on the identified areas or regions.

## Description

### FIELD OF THE INVENTION

The present invention relates to the field of medical imaging, and in particular, to the control of scanning operations by medical imaging devices.

### BACKGROUND OF THE INVENTION

Fast and high-quality medical imaging is becoming of increasing importance in the medical field, to reduce impact on clinician's time without affecting the quality of information that is usable for diagnosing or assessing a subject, i.e. a subject. Examples of medical imaging mechanisms include magnetic resonance (MR) imaging, positron emission tomography (PET) imaging techniques, X-ray imaging (including computed tomography (CT) imaging approaches) and ultrasound imaging.

In these imaging approaches, a broad range of protocols are employed in order to address different clinical questions and demands.

By way of example, for performing MR imaging on the heart, multi-slice MR scans are a common choice. Multi-slice MR is an imaging technique in which the repetition period is utilized for acquiring additional slices in other layers or planes, in differentiation for 2D techniques where every repetition period is used for single slice. The maximum number of slices of a pulse sequence depends on the repetition time, while the slice thickness is usually rather large which poses a substantial challenge for an assessment by a radiologist or an advanced image processing algorithm.

There is an ongoing desire to improve the speed of imaging acquisition, whilst preserving sufficient quality for accurately assessing the condition of the subject.

### SUMMARY OF THE INVENTION

The invention is defined by the claims.

According to examples in accordance with an aspect of the invention, there is provided a computer-implemented method of controlling a scanning operation of a medical imaging device configured to generate image data for a target subject.

The computer-implemented method comprises: obtaining initial image data of the target subject and/or a group of one or more other subjects sharing at least one characteristic with the target subject; processing the initial image data to identify one or more desired anatomical regions, each desired anatomical region being a region that has a predicted likelihood, beyond a predetermined threshold, of having one or more abnormalities, pathologies and/or diseases; and controlling a scanning operation of the medical imaging device to obtain high-resolution image data only when the desired anatomical regions of the target subject are being imaged by the medical imaging device.

The present disclosure proposes a mechanism for controlling where high-resolution imaging data is acquired. In particular, high-resolution imaging data is obtained only for anatomical regions that have a high likelihood of having one or more abnormalities, pathologies or diseases (e.g. as predicted by processing initial image data).

The initial image data comprises any image data of the target subject, or of one or more similar subjects, that is suitable for assessing whether an anatomical region may have such an abnormality, pathology or disease.

The proposed approach thereby limits high-resolution imaging to only those portions of the target subject that are likely to aid a clinician in assessing a condition of the subject, i.e. those portions that a clinician will look at when investigating the condition of the subject.

The initial image data comprises image data of a region of interest, such as the heart or other anatomical structure of a target subject.

High resolution image data is relative to the imaging capabilities of the medical imaging device. In particular, if a medical imaging device is capable of capturing image data from a lowest possible resolution to a highest possible resolution, high resolution image data may be image data captured at no less than a first predetermined percentage of the highest possible resolution, e.g. no less than 80%, no less than 90% or no less than 95% of the highest possible resolution. Low resolution image data may be image data captured at no more than a second predetermined percentage of the highest possible resolution, where the second predetermined percentage may be identical to the first predetermined percentage or a different value, e.g. no more than 50%, no more than 60% or no more than 80%.

The method may comprise obtaining first image data of the target subject, the first image data being generated during a first scanning operation of the target subject using the medical imaging device; and identifying, for each desired anatomical region, a part of the first image data that represents said desired anatomical region; wherein the step of controlling the scanning operation comprises controlling the scanning operation based on each identified part of the first image data.

First image data may, for instance, comprise image data generated during a survey or scouting scan of the target subject, a common operation during medical imaging processes. This approach facilitates identification of the relative subject-specific position of the desired anatomical regions and thereby facilitates control of the scanning operation.

The first image data may, for instance, comprise image data generated during a first pass of the target subject using the medical imaging device, i.e. earlier during the same scanning session that is to include the scanning operation, i.e. earlier during an ongoing or current scanning session. In this embodiment, the high resolution image data may effectively be image data that recaptures parts of the subject's anatomy already represented by the first image data.

The first image data preferably has a lower resolution than the high-resolution image data. Preferably, the first image data contains image data of other parts of the target subject, i.e. includes more image data of the target subject than just the desired anatomical region(s). Thus, the first image data may contain contextual anatomical areas for assisting in the assessment of the desired anatomical areas.

The method may comprise generating a visual representation, for a user interface, of the first image data and the high-resolution image data. The step of generating the visual representation may comprise: generating an image of the target subject from the first image data; and generating an image of the desired anatomical regions using the high-resolution data.

The image of the desired anatomical regions may be positioned to overlay the image of the target subject from the first image data.

The initial image data may consist of the first image data. This approach proposes a dynamic scanning operation, in which areas of concern (i.e. the desired anatomical regions) are derived based on acquired subject-specific data. This approach allows a scanning protocol to be adapted immediately after an initial scan of the target subject.

The initial image data may comprise historic image data of the target subject. In this way, the scanning operation could be configured before starting any scanning on the subject, e.g. in advance, using information from previous scans. In particular, subject-specific available information can be used. This approach may facilitate improved speed of scanning, e.g. as there is no need to process scan data partway through a scanning operation, and/or allows increased flexibility during the scanning.

The historic image data may be image data obtained by a different medical imaging device and/or during a different imaging session for the medical imaging device.

In some examples, the initial image data comprises image data of a group of a plurality of other subjects sharing at least one characteristic with the target subject. In this way, previous scans from an image data base can be analyzed to find general areas of anatomical interest for a specific subject cohort which can e.g. then be collected in an anatomical atlas. This information could then be used to assess or identify the desired anatomical regions.

The step of controlling the scanning operation may comprise controlling the scanning operation to acquire high-resolution image data of the desired anatomical regions and low-resolution image data that represents at least one or more other scanned regions of the target subject, the low-resolution image data having a lower image resolution than the high-resolution image data. The scanning operation could therefore be effectively divided into multiple scans with different resolutions which can be later combined into one complete scan.

The step of processing the initial image data to identify one or more desired anatomical regions may be performed using a machine-learning method. In this way, deep learning networks such as U-Nets or Mask R-CNN, could be used to learn anatomical and high risk regions in an end-to-end fashion.

Preferably, the initial image data and the high-resolution image data comprises magnetic resonance image data. Embodiments are particularly useful for MR imaging, as MR imaging facilitates a high degree of control over the position of areas that are imaged, meaning that a step of controlling a scanning operation is simplified.

There is also proposed a computer program product comprising computer program code means which, when executed on a computing device having a processing system, cause the processing system to perform all of the steps of any herein described method.

There is also proposed a processing system for controlling a scanning operation, configured to generate high-resolution image data for a target subject. The processing system is configured to: obtain initial image data of the target subject and/or a group of one or more other subjects sharing at least one characteristic with the target subject; process the initial image data to identify one or more desired anatomical regions, each desired anatomical region being a region that has a predicted likelihood, beyond a predetermined threshold, of having one or more abnormalities, pathologies and/or diseases; and control a scanning operation of the medical imaging device to obtain high-resolution image data only when the desired anatomical regions of the target subject are being imaged by the medical imaging device.

The skilled person would be readily capable of modifying the processing system to carry out any herein described method, and vice versa. Similarly, the skilled person would be capable of modifying the computer program product to cause a processing system (executing the computer program product) to perform any herein described method.

There is also proposed an imaging system comprising: a processing system herein described; and the medical imaging device configured to perform the scanning operation, wherein the scanning operation is controlled by the processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a generic method according to an embodiment;
Fig. 2 illustrates a method according to an embodiment;
Fig. 3 illustrates a method according to an embodiment;
Fig. 4 conceptually illustrates a process for identifying desired and contextual anatomical regions in an image;
Fig. 5 illustrates a method according to an embodiment;
Fig. 6 illustrates a processing system; and
Fig. 7 illustrates an imaging system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The present disclosure proposes an approach for adapting the acquisition protocol (i.e. scanning operation) of a medical imaging device so that anatomical areas of interest are scanned with a higher image quality than other anatomical regions. Initial image data is processed to identify relevant anatomical areas or regions, by identifying a likelihood that these areas contain a pathological condition/symptom. Using various image processing and deep learning techniques, there are multiple options to identify relevant regions in the scan area. The scanning operation is controlled based on the identified areas or regions.

The relevant anatomical regions can be detected during the course of an ongoing scanning process, which may include the scanning operation, i.e. to decide where to perform high-resolution imaging for a subject currently being scanned based on image data earlier during the same scanning process.

In some examples, previous scans of the subject are used to identify relevant regions. In some examples, scans of other (similar) subjects are used to identify the desired anatomical regions.

In the present disclosure, the terms patient and subject are used interchangeably. In the context of the present application, image data is any data that can be processed or used to generate a visually-perceptible output of (part of) a subject, and may contain raw image data, voxel data and/or display data.

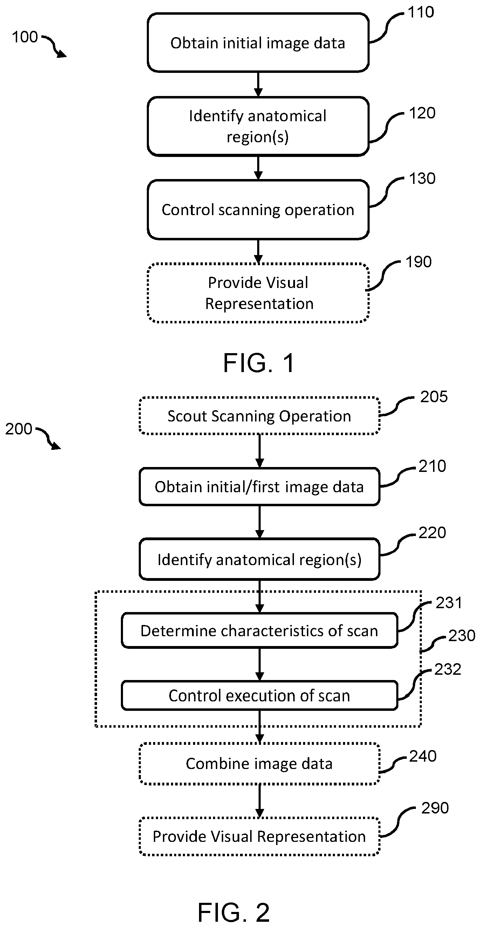

Fig. 1 illustrates a (computer-implemented) method 100 according to an embodiment. The method illustrates a generic approach to performing an embodiment of the invention, and in particular, to controlling a scanning operation of a medical imaging device. The scanning operation may form part of a larger current or ongoing medical imaging examination/session or medical imaging process being performed on the subject.

The medical imaging device is configured to generate image data for a target subject, and may consist of any suitable medical imaging device. For instance, the medical imaging device may comprise an ultrasound scanner, a magnetic resonance imaging (MRI) system, a computed tomography (CT) scanner, an x-ray scanner, a position emission tomography imaging system and so on.

Medical imaging devices may be capable of operating in at least two modes, a high-resolution imaging mode and a low-resolution imaging mode. Of course, there may be a range of other possible modes lying between these two states. For instance, an MRI system may adjust a resolution of imaging by adjusting a number of slices captured per volume area, a thickness of slices, a field of view and so on.

The method 100 may be performed by a processing system configured to communicate with and/or control the operation of a medical imaging device, such as those previously described.

The method 100 comprises a step 110 of obtaining initial image data of the target subject and/or a group of one or more subjects sharing one or more characteristics with the target subject. This initial image data may, for instance, have been captured using the same medical imaging device, a different version of the medical imaging device or a different type of medical imaging device.

The method 100 also comprises a step 120 of processing the initial image data to identify one or more desired anatomical regions. Each desired anatomical region is a region of the subject (e.g. as represented by the initial image data) that has a predicted likelihood beyond a predetermined threshold of having one or more abnormalities, pathologies and/or diseases.

In other words, step 120 comprises processing the initial image data to identify any anatomical regions that have more than predetermined likelihood of showing signs of potential pathological conditions/features. Step 120 may comprise performing an (automated) image processing technique on the initial image data, e.g. processing the initial image data using an image processing technique such as a machine-learning algorithm, to identify the desired anatomical region(s).

Step 120 may be performed, for example, by processing the initial image data using a machine-learning method. Suitable machine-learning methods for processing image data to identify potential pathological conditions/features include deep learning networks such as U-Nets, generative adversarial networks (GANs), or Mask R-CNN. A wide variety of such approaches is suggested in Ker, Justin, et al. "Deep learning applications in medical image analysis." Ieee Access 6 (2017): 9375-9389 or Yi, Xin, Ekta Walia, and Paul Babyn. "Generative adversarial network in medical imaging: A review." Medical image analysis 58 (2019): 101552.. Such methodologies can be used to learn anatomical and high risk regions in an end-to-end fashion.

Of course, other image processing technqiues could be used to identify potential desired anatomical regions in image data. A wide variety of approaches for analysing image data to identify potential pathological conditions/features have been proposed, and are widely known in the prior art. Such approaches typically perform image segmentation to identify or detect potential high-risk regions of image data.

Examples of some image processing technqiues that could be used are desribed by Heimann, Tobias, and Hans-Peter Meinzer. "Statistical shape models for 3D medical image segmentation: a review." Medical image analysis 13.4 (2009): 543-563, as well as in McInerney, Tim, and Demetri Terzopoulos. "Deformable models in medical image analysis: a survey." Medical image analysis 1.2 (1996): 91-108.

The method 100 then performs a step 130 of controlling the scanning operation (of the medical imaging device) to obtain (e.g. capture or store) high-resolution image data only when the desired anatomical regions of the target subject are being imaged. This scanning operation may be labelled a "high resolution scanning operation".

Step 130 may comprise, for instance, controlling the medical imaging device to only operate in a high-resolution imaging mode when the desired anatomical region(s) are being imaged. As another example, step 130 may comprise controlling the medical imaging device to only performing imaging on the desired anatomical region(s), e.g. and not any other region, based on the identified desired anatomical regions.

In some embodiments, step 130 may be conceptually subdivided into two stages, a first stage in which a scanning operation is planned and a second stage in which the scanning operation is executed (e.g. based on the plan).

Various approaches for controlling a scanning operation are envisaged, and depend upon the nature of the medical imaging device.

By way of example, if the medical imaging device comprises a CT scanner, the step of controlling the scanning operation may comprise controlling the position of the linear gantry (on which the subject is positioned) with respect to the rotating gantry (in which the imaging sensors are positioned) so that only image data of the desired anatomical region(s) of the subject are captured (when operating in the high resolution mode).

As another example, if the medical image device comprises a (handheld) ultrasound scanner, the step of controlling the scanning operation may comprising monitoring image data captured by the ultrasound scanner and, in response to detecting a desired anatomical region in the image data, switching to a high-resolution imaging mode to captured high-resolution image data of the desired anatomical region(s). In this example, the ultrasound scanner may be switched out of the high-resolution imaging mode responsive to captured high-resolution image data not containing the desired anatomical region, e.g. when the desired anatomical region is no longer detected.

In yet another example, a medical imaging device may mount or contain a position sensor, able to detect a position of the medical imaging device with respect to the subject. Step 130 may comprise switching the medical imaging device to a high-resolution imaging mode responsive to a detected position of the medical imaging device matching a position at which image data of the desired anatomical region(s) can be captured.

The skilled person would be readily capable of using any one or any combination of the above described approaches to perform step 130, as well as being able to envisage a number of other possibilities for performing this process.

It will be appreciated that it is not essential that the initial image data and the high-resolution image data need to be captured using the same type of medical imaging device. Purely by way of example, the initial image data may be ultrasound image data and the high-resolution image data may comprise high-resolution CT image data.

In some examples, step 130 may comprise controlling the scanning operation to perform multiple high-resolution scans over the desired anatomical area, e.g. with equal or different scan protocol parameters, to get better image quality or different contrasts of the target area.

The method 100 may further comprise a step 190 of obtaining the high-resolution image data and controlling a user interface to display a visual representation of the high-resolution image data.

Preferably, the high-resolution image data and the initial image data both comprise magnetic resonance image data. The present invention recognizes that magnetic resonance imaging is particularly susceptible to long imaging times, and it would be particularly beneficial to reduce or minimize time spent performing high-resolution imaging of a subject, e.g. by restricting high-resolution imaging to only the desired anatomical areas.

Fig. 2 illustrates a (computer-implemented) method 200 according to an embodiment, where method 200 is an embodiment of the generic method 100, described in Fi Fig. gure 1, for controlling a scanning operation of a medical imaging device.

The method 200 may be performed by a processing system configured to communicate with and/or control the operation of a medical imaging device, such as those previously described.

In this embodiment, desired anatomical regions are identified based on first image data (or "low-resolution image data") produced by a first scan of the subject (e.g. a survey scan and/or a low-resolution scan) using the medical imaging device, with a subsequent scanning operation using the medical imaging device obtaining the high-resolution image data.

In particular, the first image data is data that has been previously captured or acquired by the medical image device during a current or ongoing medical imaging session, which is to include the scanning operation that captures high-resolution image data.

The method 200 again comprises a step 210 of obtaining initial image data. Here, the initial image data comprises first image data of the subject captured by the medical imaging device during a current or ongoing medical imaging session. Thus, step 210 may comprise performing a first scanning operation (of a current or ongoing medical imaging session) to capture and acquire the first/initial image data.

The method 200 then moves to a step 220 of identifying any anatomical regions that have more than a predetermined likelihood of showing signs of potential pathological conditions/features. As previously explained, this step may comprise processing the first image data using an image processing technique such as a machine-learning method (e.g. Mask R-CNN) in order to identify any desired anatomical regions.

In particular, step 220 may comprise identifying, for each desired anatomical region, a part of the first image data that represents said desired anatomical region. This process may comprise performing a segmentation operation on the first image data to identify a part of the first image data that contains the desired anatomical region(s).

The method then moves to a step 230 of controlling the (high-resolution) scanning operation (of the medical imaging device) to obtain (e.g. capture or store) high-resolution image data only when the desired anatomical regions of the target subject are being imaged. In this embodiment, the scanning operation is controlled based on each identified part of the first image data. Thus, the presence of any representations of the desired anatomical regions in the first image data is used to control the scanning operation.

The first image data may have a lower resolution than the high-resolution image data, so that the first image data could be alternatively labelled "low-resolution image data". This means that high-resolution image data of the most relevant regions can be acquired, whilst allowing low-resolution image data of other regions to also be acquired (e.g. for improved contextual understanding without affecting diagnostic/assessment relevance).

For instance, if the image data comprises voxel data, the first image data may (per unit volume of the subject) contain no more than half the number of voxels of the high-resolution image data, e.g. no more than a quarter of the number of voxels.

In one example, step 230 comprises a step 231 of determining or setting characteristics of the scanning operation based on the identified anatomical regions (e.g. and/or the identified parts of the first image data).

For instance, for planning a scanning operation for an MRI system, step 231 may comprise selecting or calculates the scan geometry which can, for example, comprise the best field of view (FOV), the in-plane resolution, the slice thickness and the number of additional slices to be acquired during the scanning operation. As one example, for the scanning operation, step 231 may comprise configured an MRI system for capturing additional slices in-between previously captured slices (e.g. slices of the first image data).

Other approaches for suitably planning other forms/types of imaging to obtain high-resoltion image data of a region of interest will be apparent to the skilled person.

Step 230 may further comprise a step 232 of controlling the scanning operation based on the determined or set characteristics of the scanning operation. This may comprise sending or transmitting control signals to the medical imaging device to control the operation of the medical imaging device.

Controlling the scanning operation of step 230 produces high-resolution image data of the anatomical region(s) of interest. In this context, high-resolution image data is image data that has a higher resolution than the first image data.

In some examples, step 220 and optionally step 231 (if performed) are performed at least partly during the execution of step 210, e.g. whilst the first scanning operation is being performed. This means that portions of the first image data may be processed (and optionally used to plan a subsequent scanning operation) whilst further portions of the first image data are being obtained.

The method 200 may further comprise a step 240 of combining the first image data and the high-resolution image data. This combination may take place at any stage during a conventional image processing pipeline for a medical imaging system, e.g. at a raw data stage (being the direct output of a medical imaging device, at a voxel data stage (being a stage in which image data is formed of voxels) or a display data stage (being a stage in which values for pixels/voxels of a display are set).

Step 240 may comprise, for instance, integrating the high-resolution image data into the first image data by stitching sub-volumes of voxel data together or the fusion of voxel data derived from the high-resolution image data and the voxel data derived from the first image data (e.g. with different field-of views). Thus, image data may be effectively combined together at a "voxel data" stage.

This process may comprise combining the image data using a (digital) filter, to reduce noise (e.g. in the high-resolution image data).

In some examples, step 240 may be performed by generating a visual representation, for a user interface, of the first image data and the high-resolution image data. Generating the visual representation may comprise: generating an image of the target subject from the first image data; and generating an image of the desired anatomical region(s) using the high-resolution data. The image of the desired anatomical regions may be positioned to overlay the image of the target subject from the first image data - e.g. at the appropriate position within the first image data.

Thus, the combining of the first image data and the high-resolution image data may takes place at different points in the image data pipeline, e.g. at the visual representation stage (i.e. to combine display data - so that a high-resolution image overlays a lower resolution image) or at a voxel data stage (e.g. to stitch or fuse voxel data together) or at a raw data stage.

The method 200 may also comprise a step 205 of controlling the medical imaging device to perform a scout scanning operation. A scout scanning operation comprises performing a medical imaging scan to acquire information about the target subject's anatomy and/or to plan the first scanning operation. Scouting scanning operations are well known to the skilled person.

For the method 200 illustrated by Fig. 2, the first image data and the high-resolution image data are captured during the same medical imaging session (e.g. consecutively to one another).

The volume (of the subject) represented by the first image data may be larger, e.g. at least 2x larger, than the volume (of the subject) represented by the high-resolution image data.

The method 200 may further comprise a step 290 of obtaining (at least) the high-resolution image data and controlling a user interface to display a visual representation of the high-resolution image data. Step 290 may comprise controlling a user interface to display a visual representation of the combined high-resolution image data and the first image data.

As another working example of the method 200, the initial image data obtained in step 210 is a medical image (e.g. a slice), such as a 2D medical image, obtained using a first (e.g. low) resolution imaging mode. Step 220 may comprise analyzing the medical image to identify any desired anatomical regions, i.e. any anatomical regions that have more than a predetermined likelihood of showing signs of potential pathological conditions/features.

In this scenario, step 230 may comprise, in response to identifying one or more desired anatomical regions of interest in the initial image data, controlling the medical image device to capture a medical image (of the anatomical region represented by the initial image data) using a high resolution imaging mode (i.e. to produce high resolution image data) and/or capture subsequent medical images (of the scanning operation), e.g. a predetermined number of slices, using the high resolution imaging mode.

The method could then revert back to step 210 (e.g. to obtain and assess a newly captured slice).

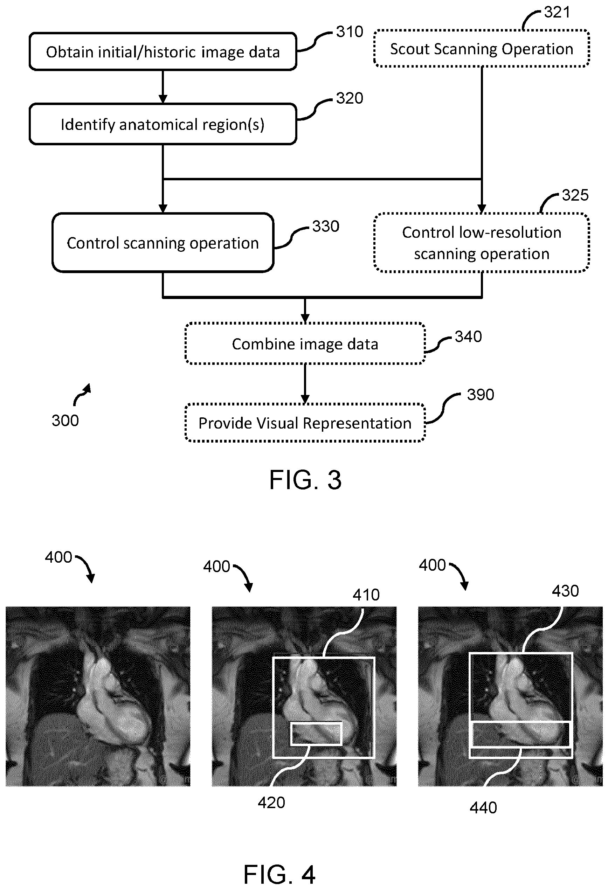

Fig. 3 illustrates a (computer-implemented) method 300 according to an embodiment, where method 200 is an embodiment of the generic method 100, described in Fig. 1, for controlling a scanning operation of a medical imaging device. The method 300 may be performed by a processing system configured to communicate with and/or control the operation of a medical imaging device, such as those previously described.

Method 300 comprises a step 310 of obtaining initial image data. Here, the initial image data comprises historic image data of the target subject, e.g. image data obtained outside of the (current or ongoing) medical imaging session that includes the scanning procedure.

The historic image data may have been captured in a previous examination or medical imaging session, if, for example the current medical imaging session is a follow-up examination. Follow up examinations are reasonably common in the medical profession, e.g. for cardiac MR to evaluate the myocardial scar and to determine how much of the border zone turned into myocardial scar.

The historic image data may be obtained from a memory or database, e.g. which stores an electronic medical record of the subject.

The method 300 then performs a step 320 of processing the historic image data (i.e. the initial image data) to identify one or more desired anatomical regions, each desired anatomical region being a region that has a predicted likelihood, beyond a predetermined threshold, of having one or more abnormalities, pathologies and/or diseases.

The process performed in step 320 may be identical to steps 120 or 220 previously described, excepting that historic image data is used (e.g. rather than recently acquired first image data).

The method may then perform a step 330 of controlling the (high-resolution) scanning operation (of the medical imaging device) to obtain (e.g. capture or store) high-resolution image data only when the desired anatomical regions of the target subject are being imaged. This may be performed in a same/similar manner to step 230 previously described.

In some examples, the method comprises a further step 325 (which may be performed before, in parallel to or after the step 330) of controlling the medical imaging device to obtain low-resolution image data (which may be functionally equivalent to the first image data obtained in the embodiment described with reference to Fig. 2). In particular, step 325 may comprising controlling a low-resolution scanning operation to capture low-resolution image data of the subject.

In performed in parallel, the performance of steps 325 and 330 may comprise switching between a medical imaging device between a low-resolution mode and a high-resolution mode, depending upon the part of the subject being imaged.

In another examples, steps 325 and 330 may be performed consecutively (in any order) with the low-resolution image data containing contextual anatomical regions for the desired anatomical region(s) and optionally including the desired anatomical region(s). The high-resolution image data may include only the desired anatomical region(s).

If performed, steps 325 and 330 can be seen as performing a single large scanning operation, which is formed of one or more high resolution scanning operations (step 330) and one or more low resolution scanning operations (step 325).

In some examples, step 320 is further configured to identify areas for identifying one or more contextual anatomical regions in the target subject, where contextual anatomical regions are regions that can provide (useful, clinical) context to the desired anatomical regions, e.g. regions that contain or are adjacent to the desired anatomical region.

In some examples, step 320 may comprise identifying a desired scan area containing the desired anatomical region(s), where the scan area also contains contextual anatomical region(s) for providing context to the desired anatomical region(s).

The step 325 of obtaining low-resolution image data may comprise defining scanning characteristics based on identified contextual anatomical regions, e.g. so that the low-resolution image data contains the contextual anatomical region.

A contextual anatomical region may contain any anatomical region that provides context to the desired anatomical region, and in particular, useful clinical content.

For instance, if a desired anatomical region is a particular fraction of an anatomical structure (e.g. the heart), the contextual anatomical region may be the rest of the anatomical structure or the entire anatomical structure (as this would provide context to the desired anatomical region). As another example, if a desired anatomical region is a blockage or stenosis in an artery, the contextual anatomical region may include the rest of the artery or some predetermined length of the artery either side of the stenosis (e.g. a 10cm stretch either side).

The precise content of the contextual anatomical region may depend upon characteristics of the corresponding desired anatomical region, e.g. the type, location and/or risk of the desired anatomical region.

In this way, information about the relevant regions (desired anatomical regions and/or contextual anatomical regions) could be used to control the performance of multiple scanning procedures (or subparts of a single longer scanning procedure), which can be later integrated into image data. For example, several multi-slice scans of distinct regions are acquired with an adapted resolution, and later combined into a single volume.

This approach effectively proposes a "focus plus context" imaging technique.

The method may further comprise a step 340 of combining the low-resolution image data and the high-resolution image data. Mechanisms for performing this process have been previously described with reference to Fig. 2.

The method 300 may further comprise a step 321 of controlling the medical imaging device to perform a scouting scanning operation. The scouting scanning operation generates scout image data, as is well known in the art.

Step 330 (and/or step 325 if performed) may comprise registering the historic image data to the scout image data, using well known image registration techniques such as rigid or deformable registration approaches or machine-learning method (e.g. U-Net based models) to identify corresponding anatomical regions. The identified anatomical regions (in the historic image data) can therefore be identified in the scout image data. This information facilitates planning of the scanning operation based on the current subject's anatomy.

A variety of image registration techniques are suggested by Oliveira, Francisco PM, and Joao Manuel RS Tavares. "Medical image registration: a review." Computer methods in biomechanics and biomedical engineering 17.2 (2014): 73-93.

Given scout image data (sometimes called localizer image data) it is possible automatically identify a representation of the desired anatomical region(s) in the scout image data and (optionally) a representation of the contextual anatomical region(s) in the scout image data, e.g. through appropriate image registration techniques following identification of the desired (and optionally contextual) anatomical region(s) in historic image data, e.g. rigid or deformable image registration or machine-learning method.

Based on the identification of the representation of the desired (and optionally contextual) anatomical region(s) in the scout image data, it is possible to control a scanning operation to capture or acquire appropriate high-resolution image data of the desired anatomical region(s) and optionally low-resolution image data of the contextual anatomical region(s). This could be performed using a plurality of different scanning sub-operations, e.g. a single low-resolution scan and a single high-resolution scan, two low-resolution scans and a single high-resolution scan and so on.

This concept is perhaps best illustrated by Fig. 4, which conceptually illustrates a process for identifying desired/contextual anatomical regions in a scout image 400 (which is repeated 3 times).

Given a scout image 400 (i.e. scout image data), an overall scan region 410 (containing both the contextual and desired anatomical regions) as well as specifically the desired anatomical regions 420 can be identified automatically. This may be performed by registering historic image data to the scout image data (using known registration approaches) and mapping identified contextual/desired anatomical regions in the historic image data to the scout image data.

Based on the result, it is possible to calculate different types of scanning areas based on the scout image, e.g. high-resolution and low-resolution scanning areas. A high-resolution scanning area 430 should include at least the desired anatomical region(s) and a low-resolution scanning area 440 should include the contextual anatomical regions.

The scanning operation can be controlled so that high resolution image data is captured when scanning the high resolution scanning area (i.e. only when imaging the desired anatomical area(s)), e.g. by controlling the medical imaging device to operate in a high resolution imaging mode. The scanning operation can also be controlled so that low resolution image data is captured when scanning the low resolution scanning area (i.e. when not imaging the desired anatomical area(s)). As previously explained, a scanning operations, could be divided into multiple sub-operations, where the medical imaging device is able to switch scanning mode between the different sub-operations.

In the illustrated example, this calculation identifies a single high-resolution area and two low-resolution areas. Depending on the protocol, these could be acquired e.g. as three separate scans, or one low-resolution scan, followed by a high-resolution scan.

Turning back to Fig. 3, the method 300 may further comprise a step 390 of obtaining (at least) the high-resolution image data and controlling a user interface to display a visual representation of the high-resolution image data. Of course, step 390 may also control the user interface to display a visual representation of the low-resolution image data (if obtained), e.g. by providing a visual representation of the combined high-resolution and low-resolution image data.

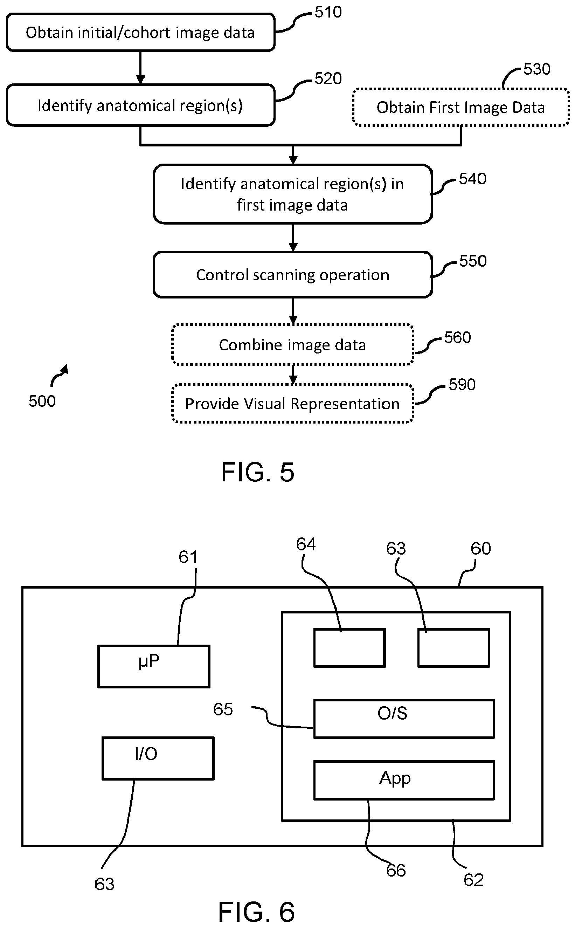

Fig. 5 illustrates another (computer-implemented) method 500 according to an embodiment, where method 500 is an embodiment of the generic method 100, described in Fig. 1, for controlling a scanning operation of a medical imaging device. The method 500 may be performed by a processing system configured to communicate with and/or control the operation of a medical imaging device, such as those previously described.

The method 500 makes use of image data ("cohort image data") of a group of a plurality of other subjects ("specific subject cohort") sharing at least one characteristic with the target subject. In other words, method 500 makes use of image data of a specific subject cohort, being a cohort of subjects, including the target subject, that share some predetermined characteristic(s).

The specific subject cohort may include (e.g. only) subjects sharing one or more of the following characteristics: a same diagnosis or disease, a same symptom, a same sign, a same age (e.g. category), a same gender, a same weight (e.g. category), a same prognosis and so on. Preferably, the specific subject cohort includes (e.g. only) subjects sharing a same diagnosis or disease, for improved contextual knowledge.

This cohort image data can be analyzed to find general areas of anatomical interest for the specific subject cohort, which could be stored as information in an anatomical atlas. This information can then be subsequently used to identify corresponding desired anatomical regions of a subject under investigation.

Method 500 comprises a step 510 of obtaining initial image data. Here the image data comprises the cohort image data previously described.

Method 500 comprises a step 520 processing the initial image data to identify one or more desired anatomical regions, each desired anatomical region being a region that has a predicted likelihood, beyond a predetermined threshold, of having one or more abnormalities, pathologies and/or diseases. Here, the method 500 comprises processing the cohort image data to identify desired anatomical regions of the cohort.

Step 520 can be performed by applying one or more image processing techniques, such as machine-learning algorithms, to the cohort image data, e.g. to identify desired anatomical regions.

In some instances, the cohort image data contains multiple instances of subject image data (e.g. for different subjects). Step 520 may comprise applying one or more machine-learning algorithms to each instance of subject image data to identify desired anatomical regions for each subject of the cohort. Desired anatomical regions of the cohort may then be selected as any desired anatomical regions that are present in more than a predetermined percentage (e.g. more than 80% or more than 90%) of the instances of subject image data.

In some examples, the cohort data may be used to train a machine-learning algorithm (e.g. for processing image data of the target subject). In particular, the cohort image data may comprise annotated image data, where the annotations are used to train the machine-learning algorithm, each annotation preferably indicating whether or not a dysfunction or pathology is identified.

Method 500 also comprises a step 530 of obtaining first image data of the target subject. The first image data may, for instance, be scout image data (obtained following a conventional or routine scout scan of the target subject). In other examples, the first image data comprises low-resolution image data.

Thus, step 530 may comprise controlling the medical imaging device to capture first image data and to obtain the captured first image data (from the medical imaging device).

The method 500 then performs a step 540 of identifying the location of the desired anatomical regions (of the cohort) in the first image data. This can be performed using any suitable identification technique, such as any suitable image processing technique like a segmentation algorithm or machine-learning method, such as a Mask R-CNN mechanism. For instance, a machine-learning algorithm trained on the cohort data may be used to process the first image data.

The method 500 then moves to step 550 of controlling a (high-resolution) scanning operation. This can be performed using any previously described approach.

Of course, it will be appreciated that steps 520, 540 may be adapted to comprise identifying (in step 520) one or more contextual anatomical regions in the cohort image data and identifying (in step 540) the location of the contextual anatomical region(s) in the first image data. This approach used may follow the approach used to identify desired anatomical regions.

Step 550 may be adapted to comprise controlling the scanning operation to obtain low-resolution image data of the contextual anatomical region(s), e.g. using the methodology previously described with reference to steps 320 and 325 of Figs. 3 and 4. In particular, the scanning operation may be controlled to capture low-resolution image data of the contextual anatomical region(s) and high-resolution image data of the desired anatomical region(s).

This identification and use of contextual anatomical regions may be omitted if, for instance, the first image data obtained in step 530 comprises the low-resolution image data.

The method 500 may also comprise a step 560 of combining the high-resolution image data with the low-resolution image data (if present) and/or the first image data (if present). Mechanisms for performing a process of combining image data have been previously described with reference to Fig. 2.

The method 500 may also comprise a step 590 of obtaining the high-resolution image data and controlling a user interface to display a visual representation of the high-resolution image data. Step 590 may be adapted to control the user interface to display a visual representation of other image data of the target subject (e.g. the first image data or, if present, the low-resolution image data) at the user interface. The representation of the high-resolution image data may be adapted to overlay the representation of other image data of the subject.

Preceding embodiments make use of "scout image data", "low-resolution image data" and/or "high-resolution image data". These types of image data are in ascending order of resolution, so that scout image data is of a lower resolution than low-resolution image data, which is of a lower resolution that high-resolution image data. The scout image data or low-resolution image data may act as the first image data in various examples.

Approaches for controlling the resolution of image data (by controlling the parameters of a scanning operation) have been previously described, and typically depend upon the type of medical imaging being performed, as it well known to the skilled person. Generally speaking, resolution can be controlled by controlled a field of view and/or a number of images captured (by the medical image device) per unit volume of the target subject.

Various approaches for identifying desired anatomical regions have been described, and make use of first image data (acquired in a current medical imaging session), historic image data (acquired in a previous medical imaging session) and/or cohort image data.

The skilled person will appreciate that any combination of these forms of image data could be used to identify desired anatomical regions. For instance, desired anatomical regions could be identified in both first image data and historic image data, and used to control a scanning operation.

By way of further example, Fig. 6 illustrates an example of a processing system 60 within which one or more parts of an embodiment may be employed. Various operations discussed above may utilize the capabilities of the processing system 60. For example, one or more parts of a system for controlling a scanning operation of a medical imaging device may be incorporated in any element, module, application, and/or component discussed herein. In this regard, it is to be understood that system functional blocks can run on a single processing system or may be distributed over several computers and locations (e.g. connected via internet).

The processing system 60 includes, but is not limited to, PCs, workstations, laptops, PDAs, palm devices, servers, storages, and the like. Generally, in terms of hardware architecture, the processing system 60 may include one or more processors 61, memory 62, and one or more I/O devices 67 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 61 is a hardware device for executing software that can be stored in the memory 62. The processor 61 can be virtually any custom made or commercially available processor, a central processing unit (CPU), a digital signal processor (DSP), a graphics processing unit (GPU) or graphics processing-like unit, or an auxiliary processor among several processors associated with the processing system 60, and the processor 61 may be a semiconductor based microprocessor (in the form of a microchip) or a microprocessor.

The memory 62 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 62 may incorporate electronic, magnetic, optical, and/or other types of storage media. Note that the memory 62 can have a distributed architecture, where various components are situated remote from one another, but can be accessed by the processor 61.

The software in the memory 62 may include one or more separate programs, each of which comprises an ordered listing of executable instructions for implementing logical functions. The software in the memory 62 includes a suitable operating system (O/S) 65, compiler 64, source code 63, and one or more applications 66 in accordance with exemplary embodiments. As illustrated, the application 66 comprises numerous functional components for implementing the features and operations of the exemplary embodiments. The application 66 of the processing system 60 may represent various applications, computational units, logic, functional units, processes, operations, virtual entities, and/or modules in accordance with exemplary embodiments, but the application 66 is not meant to be a limitation.

The operating system 65 controls the execution of other processing system programs, and provides scheduling, input-output control, file and data management, memory management, and communication control and related services. It is contemplated by the inventors that the application 66 for implementing exemplary embodiments may be applicable on all commercially available operating systems.

Application 66 may be a source program, executable program (object code), script, or any other entity comprising a set of instructions to be performed. When a source program, then the program is usually translated via a compiler (such as the compiler 64), assembler, interpreter, or the like, which may or may not be included within the memory 62, so as to operate properly in connection with the O/S 65. Furthermore, the application 66 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 67 may include input devices such as, for example but not limited to, a mouse, keyboard, scanner, microphone, camera, etc. Furthermore, the I/O devices 67 may also include output devices, for example but not limited to a printer, display, etc. Finally, the I/O devices 67 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 67 also include components for communicating over various networks, such as the Internet or intranet.

If the processing system 60 is a PC, workstation, intelligent device or the like, the software in the memory 62 may further include a basic input output system (BIOS) (omitted for simplicity). The BIOS is a set of essential software routines that initialize and test hardware at startup, start the O/S 65, and support the transfer of data among the hardware devices. The BIOS is stored in some type of read-only-memory, such as ROM, PROM, EPROM, EEPROM or the like, so that the BIOS can be executed when the processing system 60 is activated.

When the processing system 60 is in operation, the processor 61 is configured to execute software stored within the memory 62, to communicate data to and from the memory 62, and to generally control operations of the processing system 60 pursuant to the software. The application 66 and the O/S 65 are read, in whole or in part, by the processor 61, perhaps buffered within the processor 61, and then executed.

When the application 66 is implemented in software it should be noted that the application 66 can be stored on virtually any processing system readable medium for use by or in connection with any processing system related system or method. In the context of this document, a processing system readable medium may be an electronic, magnetic, optical, or other physical device or means that can contain or store a processing system program for use by or in connection with a processing system related system or method.

The application 66 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The processing system readable medium can be, for example but not limited to, an electronic, magnetic, optical, electromagnetic, infrared, or semiconductor system, apparatus, device, or propagation medium.

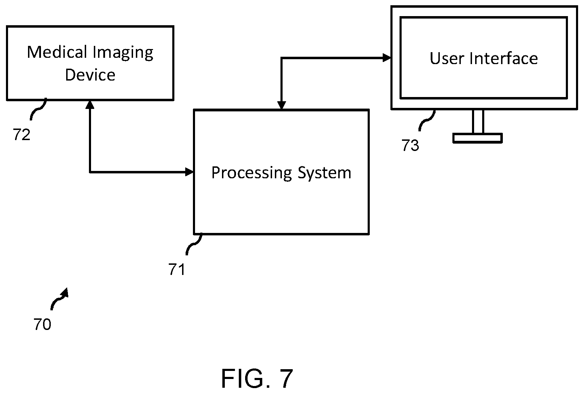

Fig. 7 illustrates an imaging system 70 according to an embodiment of the invention. The imaging system 70 comprises a processing system 71 and a medical imaging device 72.

The processing system 71 is a processing system configured to perform any herein described method. The processing system 71 is configured to communicate with the medical imaging device in order to control an operation of the medical imaging device and/or to obtain image data from the medical imaging device.

The medical imaging device 72 is configured to capture and/or acquire image data of a target subject (not shown). The medical imaging device may comprise any suitable medical imaging device whose operation can be controlled in order to acquire high-resolution imaging data.

For instance, the medical imaging device may be operable in at least two modes, a low-resolution imaging mode and a high-resolution imaging mode, where image data captured in the high-resolution imaging mode has a greater resolution than image data captured in the low-resolution imaging mode.

The medical imaging device 72 may comprise an ultrasound scanner, a magnetic resonance imaging (MRI) system, a computed tomography (CT) scanner, an x-ray scanner, a position emission tomography imaging system and so on.

The imaging system 70 may further comprise a user interface 73. The processing system 71 may be configured to control the user interface to provide a visual representation (i.e. a user-perceptible output) of at least the high-resolution image data. The processing system 71 may also control the user interface to provide a visual representation of any other image data of the target subject generated by the medical imaging device, e.g. low-resolution image data, wherein the visual representation of the high-resolution image data may overlay the visual representation of the other image data at the user interface, e.g. by positioning the representation of the high-resolution image data at the appropriate position.

Various embodiments makes use of a machine-learning algorithm to analyze and/or process image data to identify desired anatomical regions.

A machine-learning algorithm is any self-training algorithm that processes input data in order to produce or predict output data. Here, the input data comprises image data (e.g. an image, voxel data or the like) and the output data comprises desired anatomical regions (e.g. representation of the same in the image data or an identification of the desired anatomical regions).

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. In the context of the present disclosure, the training dataset may comprise (annotated) cohort image data, or other annotated image data. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example instances of image data, e.g. from cohort image data. The training output data entries correspond to desired anatomical regions (in the instances of image data), being anatomical regions that have a predicted likelihood, beyond a predetermined threshold, of having one or more abnormalities, pathologies and/or diseases.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (100, 200, 300, 500) of controlling a scanning operation of a medical imaging device (72) configured to generate image data for a target subject, the computer-implemented method comprising:
obtaining (110, 210, 310, 510) initial image data of the target subject and/or a group of one or more other subjects sharing at least one characteristic with the target subject;
processing (120, 220, 320, 520) the initial image data to identify one or more desired anatomical regions, each desired anatomical region being a region that has a predicted likelihood, beyond a predetermined threshold, of having one or more abnormalities, pathologies and/or diseases; and
controlling (130, 230, 330, 550) a scanning operation of the medical imaging device to obtain high-resolution image data only when the desired anatomical regions of the target subject are being imaged by the medical imaging device.

2. The computer-implemented method of claim 1, further comprising:
obtaining first image data of the target subject, the first image data being generated during a first scanning operation of the target subject using the medical imaging device;
identifying, for each desired anatomical region, a part of the first image data that represents said desired anatomical region;
wherein the step of controlling the scanning operation comprises controlling the scanning operation based on each identified part of the first image data.

3. The computer-implemented method of claim 2, wherein the first image data has a lower resolution than the high-resolution image data.

4. The computer-implemented method of any of claims 2 to 3, further comprising generating a visual representation, for a user interface, of the first image data and the high-resolution image data.

5. The computer-implemented method of claim 4, wherein the step of generating the visual representation comprises:
generating an image of the target subject from the first image data; and
generating an image of the desired anatomical regions using the high-resolution data.

6. The computer-implemented method of claim 5, wherein the image of the desired anatomical regions are positioned to overlay the image of the target subject from the first image data.

7. The computer-implemented method of any of claims 2 to 6, wherein the initial image data consists of the first image data.

8. The computer-implemented method of any of claims 1 to 6, wherein the initial image data comprises historic image data of the target subject.

9. The computer-implemented method of any of claims 1 to 6 or claim 8, wherein the initial image data comprises image data of a group of a plurality of other subjects sharing at least one characteristic with the target subject.

10. The computer-implemented method of any of claims 1 to 9, wherein the step of controlling the scanning operation comprises controlling the scanning operation to acquire high-resolution image data of the desired anatomical regions and low-resolution image data that represents at least one or more other scanned regions of the target subject, the low-resolution image data having a lower image resolution than the high-resolution image data.

11. The computer-implemented method of any of claims 1 to 10, wherein the step of processing the initial image data to identify one or more desired anatomical regions is performed using a machine-learning method.

12. The computer-implemented method of any of claims 1 to 11, wherein the initial image data and the high-resolution image data comprises magnetic resonance image data.

13. A computer program product comprising computer program code means which, when executed on a computing device having a processing system, cause the processing system to perform all of the steps of the method according to any of claims 1 to 12.

14. A processing system (60, 70) for controlling a scanning operation, configured to generate high-resolution image data for a target subject, the processing system being configured to:
obtain (110, 210, 310, 510) initial image data of the target subject and/or a group of one or more other subjects sharing at least one characteristic with the target subject;
process (120, 220, 320, 520) the initial image data to identify one or more desired anatomical regions, each desired anatomical region being a region that has a predicted likelihood, beyond a predetermined threshold, of having one or more abnormalities, pathologies and/or diseases; and
control (130, 230, 330, 550) a scanning operation of the medical imaging device to obtain high-resolution image data only when the desired anatomical regions of the target subject are being imaged by the medical imaging device.

15. An imaging system comprising:
a processing system according to claim 14; and
the medical imaging device configured to perform the scanning operation, wherein the scanning operation is controlled by the processing system.
